Europäisches Patentamt

European Patent Office

Office européen des brevets

(11) Publication number: **0 404 361 A1**

# EUROPEAN PATENT APPLICATION

(21) Application number: **90305743.8**

(22) Date of filing: **25.05.90**

(51) Int. Cl.5: **A61M 39/00**

(30) Priority: **21.06.89 US 369383**

(43) Date of publication of application:
**27.12.90 Bulletin 90/52**

(84) Designated Contracting States:
**DE ES FR GB IT**

(71) Applicant: **THE BOC GROUP, INC.**
**575 Mountain Avenue**
**Murray Hill New Jersey 07974(US)**

(72) Inventor: **Mashak, James E.**
**901 Lori Lane**
**Sun Prairie, Wisconsin 53590(US)**

(74) Representative: **Manitz, Gerhart, Dipl.-Phys.**
**Dr. et al**
**MANITZ, FINSTERWALD & ROTERMUND**
**Robert-Koch-Strasse 1**
**D-8000 München 22(DE)**

(54) Antidisconnect device for medical hoses.

(57) An antidisconnect device 10 is provided to prevent the inadvertent disconnection between a medical hose tubing and its male connector. The device 10 attaches to an end fitting of the medical hose and has preferably two flexible jaws 26 that are integrally moulded to the device 10 as a single, injection moulded unit. The flexible jaws 26 obtain their flexibility from the plastic composition and design and have distal ends 28 with lips 40 that normally grip the groove of a standard male fitting 18 to prevent inadvertent disconnection. The flexible jaws 26 are pivoted and biased such that they may be easily manipulated by a user to open the flexible jaws about the male fitting 18 for removal of the medical hose therefrom.

FIG. 3

# ANTIDISCONNECT DEVICE FOR MEDICAL HOSES

Medical equipment frequently utilises hoses for transmitting gases and, specifically, such medical hoses are utilised for patient hoses where the patient inhales and/or exhales through such hoses. Typical of such hoses are those connected to an anaesthesia machine and which thus supply an oxygen containing gas to the patient with an amount of an anaesthetic. Such hoses are generally intended to be disposable or are cleanable so as to minimise cross-contamination between patients. There are thus a number of fittings for connecting and disconnecting the breathing gas hoses to an anaesthesia machine or to a patient device such as a mask.

Obviously, such connections need to be positive since an inadvertent disconnection could prove to be harmful to the patient as the supply of breathing air may be discontinued. On the other hand, it is desirable to make disconnection relatively easy for personnel to remove the medical hoses for cleaning or disposal.

At the present, various standard connections for medical breathing hoses have been internationally adopted and for hoses, typically an end fitting is a 22mm rubber or plastic end fitting that is forced over a 22mm male tapered fitting on the machine or manifold. The male fitting is also standardised and includes a shoulder formed by a reduction in diameter of the fitting.

Accordingly, it is desirable to provide an antidisconnect device that is inexpensive and yet is compatible, to the extend possible, with present standard medical hose end fittings and male fittings so that extensive modifications in existing equipment need not be made and yet the device must provide a sure, positive connection of the medical hose to the male fitting. As a further feature, it is desirable that the user be able to readily disconnect the medical hose simply for disposing of that hose, preferably with a minimum of manual dexterity.

It is an aim of the present invention to provide an antidisconnect device to prevent inadvertent disconnection of a medical hose from its male fitting.

The device is adaptable to be used with standard medical hoses and male fittings.

According to the present invention there is provided a single piece injection moulded plastic antidisconnect device for preventing the inadvertent disconnection between a tapered, grooved medical hose end fitting and a standard medical male fitting having a circumferential shoulder, said device being characterised by an annular ring of predetermined inside diameter, said inside diameter being approximately the same dimension as the inside diameter of the groove in the medical hose end fitting such that said annular ring can be slid over said medical hose end fitting, and fit within and be firmly retained in the groove of said medical hose end fitting; at least one flexible jaw formed integrally in said moulded antidisconnect device said at least one flexible jaw having a proximal end and a distal end, a lip depending inwardly from the distal end of said flexible jaw to grip said circumferential shoulder when said medical hose end fitting is attached to said standard medical male fitting; said at least one flexible jaw being integrally pivoted to said antidisconnect device at a point intermediate said proximal and distal ends, such that said at least one flexible jaw can be manually pivoted by a force applied to said proximal end to disengage said lip from said circumferential shoulder for removal of said medical hose end fitting from said standard medical male fitting.

The antidisconnect device comprises an annular ring that is of predetermined dimensions and flexibility such that it slides over the tapered hose end fitting and snaps within a circular groove, easily formed in such standard hose end fittings. Once the annular ring fits within such circular groove, the antidisconnect device is firmly affixed to the hose end fitting and cannot readily be removed therefrom.

The antidisconnect device further includes at least one, preferably two flexible jaws, integrally moulded as one piece into the antidisconnect device and which jaws are pivotably with respect to the remaining portion of the moulded device. The resilience of the moulded plastic is such that the flexible jaw 8 or jaws can be pivoted by a user and, when no longer grasped by the user, the jaws are normally biased back to their original condition.

At the distal end of the flexible jaw a lip is formed that engages the circumferential shoulder formed on the male fitting when the medical hose in in its fully connected position affixed to the male fitting. Thus the antidisconnect device positively grasps the male fitting and the hose cannot inadvertently become disconnected.

When disconnection is desired, however, the user can easily disengage the lip of the flexible jaw from the shoulder and the hose is readily removed for disposal.

Thus, an antidisconnect device is produced that is a one piece moulded plastic construction and which is easily added to an existing standard medical hose end fitting and which firmly grasps a standard male fitting when the medical hose is connected thereto. Inadvertent disconnection is thereby prevented, however the antidisconnect device may be hand manipulated for easy disconnection of the hose when desired.

An embodiment of the invention will now be described by way of example, reference being made to the Figures of the accompanying diagrammatic drawings in which:-

Figure 1 is an isometric view of the antidisconnect device which is the subject matter of the invention shown connecting a medical gas hose to a male fitting;

Figure 2 is an isometric view of the antidisconnect device of Figure 1 with the medical gas hose removed from the male fittings;

Figure 3 is a side cross-sectional view of the antidisconnect device and the medical hose end fittings; and

Figure 4 is a side view, partly in cross-section, showing the manual manipulation of the antidisconnect device.

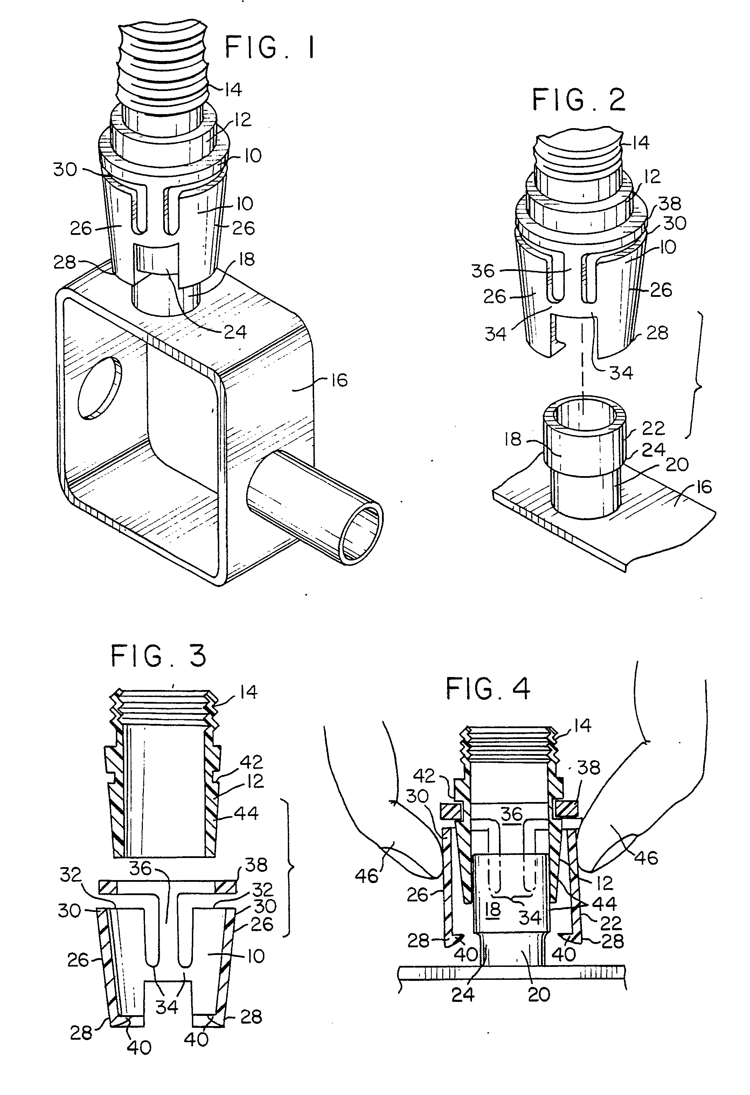

In Figures 1 and 2 there is shown an antidisconnect device 10 constructed in accordance with the present invention. The device 10 is shown in Figure 1 affixed to a medical hose end fitting 12 from which extends the medical hose 14. As stated, the medical hose 14 is normally utilised for various purposes, one in particular being the delivery to the patient of a breathing gas as well as the removal of exhaled gases from the patient.

The medical hose end fitting 12 is preferably an industry standard, one of which is a 22mm rubber or plastic medical hose end fitting. As shown in Figure 1, the antidisconnect device 10 is connected to a manifold 16 having an industry standard male fitting 18 that is usable with the subject invention. As shown more clearly in Figure 2, the standard male fitting 18 includes a reduced diameter portion 20 and enlarged diameter portion 22, thereby forming a shoulder 24 therebetween. Such construction is normal on a 22mm male fitting and includes a slight inward taper to the enlarged diameter portion 22 in the direction of its outward end.

Continuing with Figures 1 and 2, the antidisconnect device 10 is a unitary, plastic injection moulded part for ease and economical production on a large scale basis and is preferably made of a plastics material such as Delran, however other plastics may be used. It is advantageous that such plastics be steam autoclavable.

In the preferred embodiment, two flexible jaws 26 are formed in the antidisconnect device 10, opposite each other and each have a distal end 28 extending away from medical hose end fitting 12. A U-shaped slot 32 is formed in antidisconnect device 10 to form each of the flexible jaws 26 forming a pivot point 34 joining the flexible jaws 26 intermediate their proximal and distal ends 30 and 28 to a web 36. Extending from web 36 is an annular ring 38 of predetermined dimensions as will later be explained. At the distal ends 28 of each of the flexible jaws 26, there is formed a lip 40 depending inwardly.

Turning now to Figure 3, there is shown a side cross-sectional view of the antidisconnect device 10 displaced with respect to the medical hose end fitting 12. As can be noted, a circular groove 42 is readily formed in medical hose end fitting 12 of standard dimensions and can be moulded into such fittings. All other dimensions of the end fittings are standard presently used. The annular ring 38 is similarly dimensioned such that the antidisconnect device 10 may be slid onto the medical hose end fitting 12 and the annular ring 38 advances over the tapered surface 44 until it firmly snaps into the circular groove 42. Due to the predetermined dimensions and resilience of the plastics material used to mould the antidisconnect device, once the annular ring 38 has snapped into the circular groove 42, the antidisconnect device 10 is firmly affixed to the medical hose end fitting 12 and cannot be removed therefrom without extreme force.

Turning finally to Figure 4, there is shown a side view, partly in cross section, showing the hand manipulation of an antidisconnect device 10 in the removal of a medical hose 14 from a standard male fitting 18. As shown, the user is disconnecting the medical hose 14 by squeezing toward each other, the proximal ends 30 of flexible jaws 26. In the position shown, the medical hose end fitting 12 is firmly secured to standard male fitting 18, however, by the squeezing action of fingers 46, preferably thumb and forefinger, the flexible jaws 26 both pivot about their pivot points 34 and their distal ends 28 move outwardly to disengage lips 40 from the shoulder 24.

Removal of the medical hose 14 is thus easily accomplished, yet while affixed to standard male fitting 18, inadvertent removal is extremely difficult.

## Claims

1. A single piece injection moulded plastics antidisconnect device 10 for preventing the inadvertent disconnection between a tapered, grooved medical hose end fitting 12 and a standard medical male fitting 18 having a circumferential shoulder 24, said device being characterised by an annular ring 38 of predetermined inside diameter, said inside diameter being approximately the same dimension as the inside diameter of the groove 42 in the medical hose end fitting 12 such that said annular ring 38 can be slid over said medical hose end fittings 12, and fit within and be firmly retained in the groove 42 of said medical hose end fitting 12; at least one flexible jaw 26 formed integrally in said moulded antidisconnect device 10, said at least one flexible jaw 26 having a proximal end 30 and a distal end 28, a lip 40 depending inwardly from the distal end 28 of said flexible jaw 26 to grip said circumferential shoulder 24 when said medical hose end fitting 12 is attached to said standard medical male fitting 18; said at least one flexible jaw 26 being integrally pivoted to said antidisconnect device at a point intermediate said proximal and distal ends 30, 28, such that said at least one flexible jaw 26 can be manually pivoted by a force applied to said proximal end 30 to disengage said lip 40 from said circumferential shoulder 24 for removal of said medical hose end fitting 12 from said standard medical male fitting 18.

2. A single piece injection moulded plastics antidisconnect device as claimed in Claim 1, characterised in that two flexible jaws 26 are provided, located opposite each other.

3. A single piece injection moulded plastics antidisconnect device as claimed in Claim 2, characterised in that said flexible jaws 26 are formed by the formation of two U-shaped recesses 32 in said device 10.

EP 0 404 361 A1

FIG. 1

FIG. 2

FIG. 3

FIG. 4

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | GB-A-2 077 377 (CRAIG MEDICAL PRODUCTS) * Page 1, lines 87-99; page 1, lines 124-129; figure 1 * | 1,2 | A 61 M 39/00 |
| A | | 3 | |
| Y | US-A-3 245 703 (MANLY) * Column 1, lines 54-60; figures 3,4 * | 1,2 | |
| A | US-A-4 836 580 (FARRELL) * Figure 3 * | 1-3 | |
| A | US-A-4 589 684 (NOWACKI et al.) * Claims * | 1 | |
| A | US-A-4 711 636 (BIERMAN) * Figure 5 * | 1 | |

TECHNICAL FIELDS
SEARCHED (Int. Cl.5)

A 61 M
F 16 L

The present search report has been drawn up for all claims

| Place of search | Date of completion of the search | Examiner |
|---|---|---|
| THE HAGUE | 24-09-1990 | VLECK J.M. |

EPO FORM 1503 03.82 (P0401)